# EUROPEAN PATENT APPLICATION

(11) **EP 4 635 440 A1**
(43) Date of publication of application: **22.10.2025**
(21) Application number: 24223313.8
(22) Date of filing: 26.12.2024
(51) Int. Cl.: A61B 18/22, A61B 34/10

(54) **LASER ABLATION METHOD, APPARATUS AND DEVICE, AND COMPUTER READABLE STORAGE MEDIUM**

(30) Priority: 16.04.2024 CN 202410460247
(71) Applicant: Hangzhou GenLight MedTech Co., Ltd., Hangzhou, Zhejiang (CN)
(72) Inventor: CAO, Peng, Hangzhou (CN)
(74) Representative: Monteiro Alves, Inês

(57) **Abstract**

The present disclosure provides a laser ablation method, a laser ablation apparatus, a laser ablation device, a computer-readable storage medium, and a computer program product. The laser ablation method includes: determining a corresponding laser ablation mode based on real-time lesion region information at a first time; obtaining real-time lesion region information at a (i+1)th time; determining whether a conformity index between the real-time lesion region information at the (i+1)th time and expected lesion region information after an i-th laser ablation exceeds a first set threshold, to determine a (i+1)th laser ablation mode or end the laser ablation. In the present application, it is able to provide an appropriate ablation strategy according to an actual condition of a lesion and complete the ablation strategy in a single ablation operation, with relatively low risk.

## Description

### TECHNICAL FIELD

The present disclosure relates to the field of laser ablation, in particular to a laser ablation method, a laser ablation apparatus, a laser ablation device, a computer-readable storage medium, and a computer program product.

### BACKGROUND

A laser ablation technology is to irradiate the lesion region that needs to be ablated with laser light emitted by a laser ablation device, and perform fragmentation of stones, cutting of a soft tissue, or thermal coagulation denaturation of a soft tissue through a thermal effect or shock waves generated by the laser. The laser ablation technology is widely used due to the short ablation time and small incision.

In the related art, the laser ablation device can only emit one kind of laser. For a particular lesion region, it requires to change an ablation mode, which involves repeatedly plugging and unplugging of a fiber that emits the laser for replacement. This leads to a high risk of infection in a lesion region, long ablation time, and higher ablation costs.

### SUMMARY

In view of the shortcomings in the related art, the present disclosure provides a laser ablation method, a laser ablation apparatus, a laser ablation device, a computer-readable storage medium, and a computer program product, so as to address the technical issues of high infection risk in the lesion region, long ablation time, and high ablation costs in the related art.

In a first aspect, the embodiments of the present disclosure provide a laser ablation method, including:
determining a corresponding laser ablation mode based on real-time lesion region information at a first time;
obtaining real-time lesion region information at a (i+1)th time, where the (i+1)th time is later than an i-th time, and i is a positive integer;
determining whether a conformity index between the real-time lesion region information at the (i+1)th time and expected lesion region information after an i-th laser ablation exceeds a first set threshold;
determining, in response to that the conformity index between the real-time lesion region information at the (i+1)th time and the expected lesion region information after the i-th laser ablation exceeds the first set threshold, a (i+1)th laser ablation mode according to an expected laser ablation mode of a (i+1)th laser ablation in a laser ablation strategy and the real-time lesion region information at the (i+1)th time, and continuing to determine a next laser ablation mode through repeating the above steps of obtaining and subsequent determining;
ending the laser ablation in response to that the conformity index between the real-time lesion region information at the (i+1)th time and the expected lesion region information after the i-th laser ablation does not exceed the first set threshold.

Optionally, prior to determining the corresponding laser ablation mode based on the real-time lesion region information at the first time, the method further includes:
obtaining initial lesion region information of a user and a corresponding laser ablation strategy, where the laser ablation strategy includes a quantity of laser ablation, an expected laser ablation mode corresponding to each laser ablation, and expected lesion region information after each laser ablation.

Optionally, prior to obtaining the initial lesion region information of the user and the corresponding laser ablation strategy, the method further includes:
receiving digital image information of the user;
performing multimodal three-dimensional modeling based on the digital image information to obtain a three-dimensional model of the user;
receiving a marking operation for a position of a tissue in the three-dimensional model to determine the initial lesion region information of the user; where the tissue includes a lesion, nerve fiber tracts, a brain partition and a blood vessel;
receiving expected lesion region information after a target ablation of a lesion corresponding to the initial lesion region information; and
determining the laser ablation strategy based on the initial lesion region information and the expected lesion region information.

Optionally, the laser ablation method further includes:
receiving the marking operation for the position of the tissue in the three-dimensional model,
determining whether there is a non-ablation region, and
marking the non-ablation region in response to that there is a non-ablation region.

Optionally, determining the corresponding laser ablation mode based on the real-time lesion region information at the first time includes:
obtaining the real-time lesion region information at the first time; and
determining an expected laser ablation mode of a first laser ablation in the laser ablation strategy as the laser ablation mode in response to that a conformity index between the real-time lesion region information at the first time and the initial lesion region information is less than a second set threshold.

Optionally, determining the (i+1)th laser ablation mode according to the expected laser ablation mode of the (i+1)th laser ablation in the laser ablation strategy and the real-time lesion region information at the (i+1)th time includes:
adjusting, in response to that a conformity index between the expected lesion region information at the (i+1)th time in the laser ablation strategy and the real-time lesion region information at the (i+1)th time is less than a third set threshold, the expected laser ablation mode of the (i+1)th laser ablation according to the real-time lesion region information at the (i+1)th time, to obtain the corresponding laser ablation mode;
maintaining, in response to that the conformity index between the expected lesion region information at the (i+1)th time and the real-time lesion region information at the (i+1)th time is greater than or equal to the third set threshold, a laser ablation mode at the i-th time as the laser ablation mode at the (i+1)th time.

Optionally, the expected lesion region information includes: an area of a lesion region; where the area of the lesion region after the (i+1)th laser ablation is smaller than the area of the lesion region after the i-th laser ablation.

Optionally, the laser ablation mode includes at least one of a laser wavelength, an ablation timing sequence, a laser output power, a laser irradiation manner, a quantity of lasers or a laser irradiation position.

Optionally, any two laser ablation modes have different laser wavelengths and/or quantities of lasers.

Optionally, the real-time lesion region information includes real-time temperature information of a lesion region; and the laser ablation method further includes:
controlling, in response to that the real-time temperature information is greater than a preset temperature threshold, an optical fiber assembly for outputting the laser to be cooled until subsequent real-time temperature information is not greater than the preset temperature threshold.

Optionally, the preset temperature threshold is determined by the initial lesion region information.

Optionally, the laser ablation method further includes: obtaining real-time temperature information, and obtaining a three-dimensional temperature distribution map of the real-time temperature information based on the three-dimensional model of the user.

Optionally, obtaining the real-time temperature information includes:
obtaining the real-time temperature information through parallel imaging, where a scanning slice thickness is not less than 3 mm and not greater than 5 mm; or,
obtaining the real-time temperature information through three-dimensional scanning; or,
obtaining the real-time temperature information through thin slice scanning, where a scanning slice thickness is less than 3 mm.

Optionally, the real-time lesion region information includes: a plurality of pieces of target region information, and the method further includes: determining the laser ablation mode corresponding to each target region information.

Optionally, the real-time lesion region information includes boundary information of a lesion region, and the method further includes: determining in real time whether the boundary information exceeds a preset boundary threshold.

Optionally, the laser ablation method further includes: color-marking the real-time lesion region information in a three-dimensional model, where the real-time lesion region information corresponding to each time has a different marking color from the expected lesion region information.

In a second aspect, the embodiments of the present disclosure provide a laser ablation apparatus, including:
a first processing module, configured to determine a corresponding laser ablation mode based on real-time lesion region information at a first time;
a second obtaining module, configured to obtain real-time lesion region information at a (i+1)th time, wherein the (i+1)th time is later than an i-th time, and i is a positive integer; where a lesion region at the (i+1)th time is ablated based on a laser ablation mode at the i-th time; and
a second processing module, configured to determine a (i+1)th laser ablation mode according to a laser ablation strategy and the real-time lesion region information at the (i+1)th time, until a conformity index between real-time lesion region information at a subsequent time and expected lesion region information after a final laser ablation exceeds a first set threshold.

In a third aspect, the embodiments of the present disclosure provide a laser ablation device, including a control host and a computer program stored on the control host, where the control host is configured to execute the computer program to implement the steps of the laser ablation method in the first aspect.

In a fourth aspect, the embodiments of the present disclosure provide a computer readable storage medium having a computer program stored thereon, the computer program implementing, when executed by a laser ablation device, the steps of the laser ablation method in the first aspect.

In a fifth aspect, the embodiments of the present disclosure provide a computer program product, including a computer program, where the computer program implements, when executed by a control host, the steps of the laser ablation method in the first aspect.

The technical solutions in the embodiments of the present disclosure give rise to the following beneficial technical effects.

In the embodiments of the present disclosure, it is able to determine the laser ablation mode appropriate for the real-time lesion region information based on the real-time lesion region information of the user, and provide a reference for laser ablation. Furthermore, the real-time lesion region information is obtained during each laser ablation process, and the laser ablation mode appropriate for the real-time (i.e., current) lesion region is determined according to the lesion region after the real-time (i.e., current) ablation, which has strong specificity. This effectively avoids the increased infection risk of the lesion region due to repeatedly changing the ablation fiber to change the ablation mode in one ablation process and also correspondingly reduces ablation costs.

The additional aspects and advantages of the present disclosure will be given or may become apparent in the following description, or may be understood through the implementation of the present disclosure.

### BRIEF DESCRIPTION OF THE DRAWINGS

The above and/or additional aspects as well as advantages of the present disclosure will become apparent and are easily understood in the following description with reference to the following drawings. In these drawings,
Fig. 1 is a schematic flowchart illustrating a laser ablation method according to the embodiments of the present disclosure;
Fig. 2 is another schematic flowchart illustrating the laser ablation method according to the embodiments of the present disclosure;
Fig. 3 is a schematic diagram of a laser ablation apparatus according to the embodiments of the present disclosure;
Fig. 4 is a schematic diagram of a laser ablation device according to the embodiments of the present disclosure.

### DETAILED DESCRIPTION

The embodiments of the present disclosure will be described hereinafter in conjunction with the accompanying drawings. It should be appreciated that the embodiments described hereinafter with reference to the drawings are exemplary illustrations used to explain the technical solutions of the embodiments of the present disclosure, but shall not be construed as limiting the technical solutions of the embodiments of the present disclosure.

As can be appreciated by a person skilled in the art, unless otherwise defined, the singular forms "a," "an," and "the" used herein are intended to include the plural forms as well. Moreover, the terms "comprises," "comprising," "includes," and/or "including," when used in the description of the present disclosure, specify the presence of stated features, integers, steps, operations, elements, components, and/or groups thereof, but do not preclude the presence or addition of one or more other features, integers, steps, operations, elements, components, and/or groups thereof. In the case that one element is connected or coupled to another element, it may be directly connected or coupled to the other element, or an intermediate element may be arranged therebetween. At this time, the element may be connected or coupled to the other element in a wireless or wired manner. In addition, the term "and/or" used herein refers to at least one of the items defined by the term. For example, "A and/or B" can be implemented as "A," "B," or "A and B."

In order to make the embodiments, the technical solutions and the advantages of the present disclosure more apparent, the embodiments of the present disclosure will be described hereinafter in further detail in conjunction with the drawings.

Generally, in the related laser ablation technologies, one ablation fiber can only emit one kind of ablation laser. Due to the different characteristics of lesion regions, it is difficult to achieve the ablation effect through only one kind of ablation mode in one ablation process. Thus, various ablation fibers are often replaced during the one ablation process, thereby using multiple ablation modes to perform ablation on the same lesion region multiple times. Replacing the ablation fibers involves repeatedly plugging and unplugging the ablation fibers at an incision, leading to a high risk of infection at the incision and increasing the corresponding incision disinfection costs and ablation device costs.

The present disclosure provides a laser ablation method, a laser ablation apparatus, a laser ablation device, a computer-readable storage medium, and a computer program product, so as to address the above-referenced technical issues in the related art.

The following detailed description of specific embodiments of the present disclosure explains the technical solutions of the present disclosure and how they address the above-referenced technical issues. It should be noted that the following embodiments can be referenced for each other, or combined with each other. Identical terms, similar features, and similar implementation steps in different embodiments will not be repeatedly described.

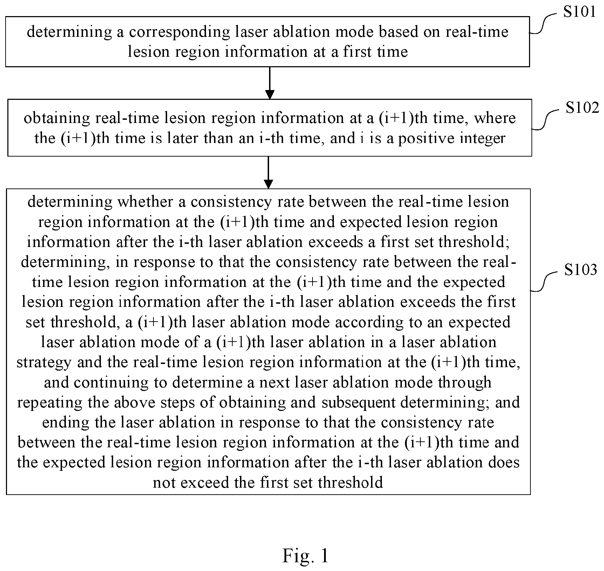

The embodiments of the present disclosure provide a laser ablation method, as shown in Fig. 1, including steps S101 to S 103.

S 101, determining a corresponding laser ablation mode based on real-time lesion region information at a first time.

S102, obtaining real-time lesion region information at a (i+1)th time, where the (i+1)th time is later than an i-th time, and i is a positive integer.

Optionally, a lesion region at the (i+1)th time is subjected to the i-th laser ablation based on the laser ablation mode at the i-th time.

S103, determining whether a conformity index between the real-time lesion region information at the (i+1)th time and expected lesion region information after the i-th laser ablation exceeds a first set threshold; determining, in response to that the conformity index between the real-time lesion region information at the (i+1)th time and the expected lesion region information after the i-th laser ablation exceeds the first set threshold, a (i+1)th laser ablation mode according to an expected laser ablation mode of a (i+1)th laser ablation in a laser ablation strategy and the real-time lesion region information at the (i+1)th time, and continuing to determine a next laser ablation mode through repeating the above steps of obtaining (i.e., S102) and determining in S103; and ending the laser ablation in response to that the conformity index between the real-time lesion region information at the (i+1)th time and the expected lesion region information after the i-th laser ablation does not exceed the first set threshold.

In the laser ablation method of the embodiments of the present disclosure, it is able to determine the laser ablation mode appropriate for the real-time lesion region information based on the real-time lesion region information of the user, and provide a reference for laser ablation. Furthermore, the real-time lesion region information is obtained during each laser ablation process, and the laser ablation mode appropriate for the real-time (i.e., current) lesion region is determined according to the lesion region after the real-time (i.e., current) ablation, which has strong specificity. This effectively avoids the increased infection risk of the lesion region due to repeatedly changing the ablation fiber to change the ablation mode in one ablation process and also correspondingly reduces ablation costs.

Optionally, in the laser ablation mode obtained first, the expected quantity of laser ablations may be set to n times, with i+1 being less than n.

Optionally, prior to S101, the method further includes: obtaining initial lesion region information of a user and a corresponding laser ablation strategy, where the laser ablation strategy includes a quantity of laser ablation an expected laser ablation mode corresponding to each laser ablation, and expected lesion region information after each laser ablation.

Optionally, prior to the step of obtaining the initial lesion region information of the user and the corresponding laser ablation strategy, the method further includes: receiving digital image information of the user; performing multimodal three-dimensional modeling based on the digital image information to obtain a three-dimensional model of the user; receiving a marking operation for a position of a tissue in the three-dimensional model to determine the initial lesion region information of the user; where the tissue includes a lesion, a nerve and a blood vessel; receiving expected lesion region information after a target ablation of a lesion corresponding to the initial lesion region information; and determining the laser ablation strategy based on the initial lesion region information and the expected lesion region information.

Optionally, the method further includes: receiving the marking operation for the position of the tissue in the three-dimensional model, determining whether there is a non-ablation region, and marking the non-ablation region in response to that there is a non-ablation region.

Optionally, S102 of determining the corresponding laser ablation mode based on the real-time lesion region information at the first time includes: obtaining the real-time lesion region information at the first time; and determining an expected laser ablation mode of a first laser ablation in the laser ablation strategy as the laser ablation mode in response to that a conformity index between the real-time lesion region information at the first time and the initial lesion region information is less than a second set threshold.

Optionally, S104 of determining the (i+1)th laser ablation mode according to the expected laser ablation mode of the (i+1)th laser ablation in the laser ablation strategy and the real-time lesion region information at the (i+1)th time includes: adjusting, in response to that a conformity index between the expected lesion region information at the (i+1)th time in the laser ablation strategy and the real-time lesion region information at the (i+1)th time is less than a third set threshold, the expected laser ablation mode of the (i+1)th laser ablation according to the real-time lesion region information at the (i+1)th time, to obtain the corresponding laser ablation mode; maintaining, in response to that the conformity index between the expected lesion region information at the (i+1)th time and the real-time lesion region information at the (i+1)th time is greater than or equal to the third set threshold, a laser ablation mode at the i-th time as the laser ablation mode at the (i+1)th time.

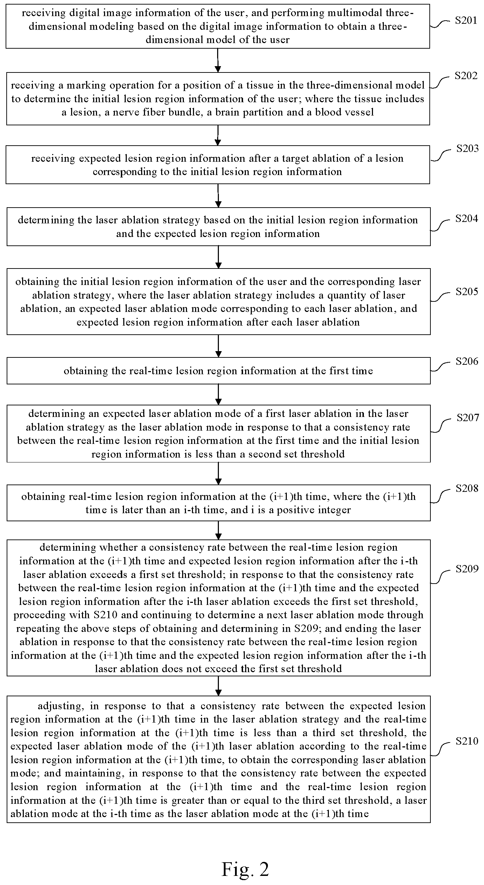

The embodiments of the present disclosure further provide another laser ablation method, as shown in Fig. 2, including steps S201 to S201.

S201, receiving digital image information of the user, and performing multimodal three-dimensional modeling based on the digital image information to obtain a three-dimensional model of the user.

Optionally, the digital imaging information may be manually inputted or obtained by an imaging device and transmitted to the laser ablation device.

S202, receiving a marking operation for a position of a tissue in the three-dimensional model to determine the initial lesion region information of the user; where the tissue includes a lesion, nerve fiber tracts, a brain partition and a blood vessel.

Optionally, the marking operation may be manually performed by technicians based on the user's digital imaging information and the user's treatment effects, thereby providing enhanced targeting capability and accuracy.

Optionally, the tissue includes a brain tissue, and the ablation position of the laser ablation is located within a cranial cavity of the user.

Optionally, the method further includes: receiving the marking operation for the position of the tissue in the three-dimensional model, determining whether the user has a non-ablation region, and marking the non-ablation region in response to that the user has a non-ablation region.

Specifically, the non-ablation region may be a small portion within the lesion region that does not require ablation operations. For example, considering that some tumor regions need to undergo biopsy in advance, after the biopsy is taken, the user's digital imaging information may be received to obtain the user's three-dimensional model, identify a region where the biopsy is removed, and make a segmentation marking, which can be displayed in three dimensions simultaneously. Next, ablation is performed, thereby reducing the impact of this non-ablation region on the calculation and evaluation process during the assessment of subsequent ablation results. In addition, it can also alert technicians to pay attention to the difference in temperature information between the non-ablation region and other locations within the lesion region.

S203, receiving expected lesion region information after a target ablation of a lesion corresponding to the initial lesion region information.

In this embodiment, the expected lesion region information after the target ablation may be determined through large sample data, measured and calculated through experiments, or determined based on the technician's experience.

Optionally, the expected lesion region information includes: an area of a lesion region, and the area of the lesion region after the (i+1)th laser ablation is smaller than the area of the lesion region after the i-th laser ablation.

In this embodiment, appropriate wavelengths of laser are selected for ablation based on the different areas of lesion regions, allowing for the rational use of laser resources and cost savings.

As can be appreciated, the total quantity of laser ablation is n times, and any interim laser ablation is referred to as an i-th ablation. The i-th laser ablation corresponds to a laser ablation at the i-th time.

S204, determining the laser ablation strategy based on the initial lesion region information and the expected lesion region information.

In this embodiment, the initial lesion region information refers to an original state of the lesion region of the user, and the expected lesion region information refers to a desired target state of the lesion region of the user. Based on the original and target states of the lesion region, the appropriate laser ablation mode, the quantity of ablation, etc., are determined. For example, a laser with one wavelength is used for coarse ablation of a lesion region with a large area, while a laser with another wavelength is used for fine ablation of a lesion region with a small area.

Optionally, the laser ablation mode includes at least one of a laser wavelength, an ablation timing sequence, a laser output power, a laser irradiation manner, a quantity of lasers or a laser irradiation position.

Optionally, any two laser ablation modes have different laser wavelengths and/or quantities of lasers.

For example, in some possible embodiments, two or more laser ablation modes are required for a particular lesion region. Each laser ablation mode may correspond to one specific laser, with a laser wavelength of a first laser ablation mode being 980 nanometers and a laser wavelength of a second laser ablation mode being 1064 nanometers.

For another example, the laser ablation modes correspond to the same laser, with both the first and second laser ablation modes having a laser wavelength of 980 nanometers, but differing in ablation duration or the timing sequence. Alternatively, the laser irradiation manners of the lasers may differ, or, the target irradiation regions of the lasers may differ, and these may be combined according to the practical needs.

For yet another example, each laser ablation mode includes at least two types of ablation lasers, e.g., one laser ablation mode having 2 types of ablation lasers and another laser ablation mode having 3 types of ablation lasers.

S205, obtaining the initial lesion region information of the user and the corresponding laser ablation strategy, where the laser ablation strategy includes a quantity of laser ablation, an expected laser ablation mode corresponding to each laser ablation, and expected lesion region information after each laser ablation.

In this step, each laser ablation causes a certain degree of change in the lesion region. Each ablation corresponds to expected lesion region information. Once predetermined ablation strategies are completed, the lesion region is expected to reach the final expected lesion region information after the last ablation in an ideal state.

S206, obtaining the real-time lesion region information at the first time.

In this step, the real-time lesion region information may be inputted by the technicians into a control host or obtained through a particular device and transmitted to the control host.

S207, determining an expected laser ablation mode of a first laser ablation in the laser ablation strategy as the laser ablation mode in response to that a conformity index between the real-time lesion region information at the first time and the initial lesion region information is less than a second set threshold.

In this step, the initial lesion region information is obtained before the ablation process. However, before proceeding with ablation, it requires to confirm whether there has been a significant change in the nature or condition of the lesion region. If not (i.e., the conformity index is less than the second set threshold), the previously determined laser ablation mode in the laser ablation strategy is used for a subsequent ablation, i.e., entering the laser ablation process. If there has been a significant change in the nature or condition of the lesion region (i.e., the conformity index is not less than the second set threshold), the originally determined laser ablation strategy is not appropriate for the current lesion region, and a new appropriate laser ablation strategy needs to be determined.

As can be appreciated, this step further includes: ending the laser ablation in response to that the conformity index between the real-time lesion region information at the first time and the initial lesion region information is greater than the second set threshold.

As can be appreciated, in this step, the laser ablation mode for each subsequent ablation is determined periodically. One period includes the following steps S208-S210, where the real-time lesion region information is periodically compared with the expected lesion region information to determine whether a change in ablation mode is required. The specific steps to determine the ablation mode are as follows.

S208, obtaining real-time lesion region information at the (i+1)th time, where the (i+1)th time is later than an i-th time, and i is a positive integer. The lesion region at the (i+1)th time is subjected to the i-th laser ablation based on the laser ablation mode at the i-th time.

At S208, the laser ablation process begins. After starting laser ablation, in this step, lesion region information is obtained in real-time at several predetermined times or according to a specific time pattern, to determine whether the ablated lesion region has achieved the pre-set ablation effect.

For example, the laser ablation strategy includes a quantity of laser ablation, an expected laser ablation mode corresponding to each laser ablation, and expected lesion region information after each laser ablation. When the quantity of laser ablation is 1, ablation may begin after obtaining the real-time lesion region information at the first time, and may end at a second time. Next, real-time lesion region information at the second time is obtained, and the real-time lesion region information at the first time is compared with the real-time lesion region information at the second time, to determine whether the real-time lesion region information at the second time has achieved the ablation effect. Specific details can be referred to in step S209.

S209, determining whether a conformity index between the real-time lesion region information at the (i+1)th time and expected lesion region information after the i-th laser ablation exceeds a first set threshold; in response to that the conformity index between the real-time lesion region information at the (i+1)th time and the expected lesion region information after the i-th laser ablation exceeds the first set threshold, proceeding with S210 and continuing to determine a next laser ablation mode through repeating the above steps of obtaining (i.e., S208) and determining in S209; and ending the laser ablation in response to that the conformity index between the real-time lesion region information at the (i+1)th time and the expected lesion region information after the i-th laser ablation does not exceed the first set threshold.

S210, adjusting, in response to that a conformity index between the expected lesion region information at the (i+1)th time in the laser ablation strategy and the real-time lesion region information at the (i+1)th time is less than a third set threshold, the expected laser ablation mode of the (i+1)th laser ablation according to the real-time lesion region information at the (i+1)th time, to obtain the corresponding laser ablation mode; and maintaining, in response to that the conformity index between the expected lesion region information at the (i+1)th time and the real-time lesion region information at the (i+1)th time is greater than or equal to the third set threshold, a laser ablation mode at the i-th time as the laser ablation mode at the (i+1)th time.

In this step, when the conformity index between the expected lesion region information at the (i+1)th time in the laser ablation strategy and the real-time lesion region information at the (i+1)th time is small, i.e., there is a large difference between the expected ablation effect after the (i+1)-th ablation and the real-time lesion region information at the (i+1)th time, it indicates that the ablation strategy is inappropriate and requires to be adjusted. When the conformity index between the expected lesion region information at the (i+1)th time in the laser ablation strategy and the real-time lesion region information at the (i+1)th time is large, i.e., there is a small difference between the expected ablation effect after the (i+1)-th ablation and the real-time lesion region information at the (i+1)th time, it suggests that the ablation process is proceeding according to the intended strategy, and it basically does not require to adjust the current ablation mode.

In the embodiments, it is able to adjust the ablation mode based on the real-time lesion region information, thereby providing enhanced targeting capability and achieving better ablation results.

As can be appreciated, subsequent to S210, the method further includes: determining whether a conformity index between the real-time lesion region information after the laser mode ablation corresponding to the (i+1)th time and the expected lesion region information after a final laser ablation exceeds a first set threshold. If yes, it means that the last real-time lesion region information has achieved the expected ablation effect, and the ablation process can be ended. If no, it returns to S209 and continues the determination operation.

As can be appreciated, there are three processes of determining the conformity index in the cyclic method in the embodiments of the present disclosure. One process of determining the conformity index is performed before the cycle, specifically to determine whether the conformity index of the real-time lesion region information at the first time and the initial lesion region information reaches the second set threshold, that is, to determine whether the real-time lesion region information at the first time is basically consistent with the initial lesion region information.

Another process of determining the conformity index is performed after the cycle, specifically, determining whether the conformity index between the final real-time lesion region information and the expected lesion region information exceeds the first set threshold. If yes, it means that the conformity index is relatively high, and the effect of this laser ablation is basically achieved, and the laser ablation is ended; if no, it means that the conformity index is relatively low, and the effect of this laser ablation has not been achieved, and the laser ablation operation will continue.

There is yet another process of determining the conformity index that is performed within the cycle, specifically, determining whether the conformity index between the real-time lesion region information at a specific time and the expected lesion region information that corresponds to this specific time reaches the third set threshold. If yes, it indicates that ablation is performed in accordance with a predetermined laser ablation mode in this cycle. If no, it indicates that it is difficult to perform ablation in accordance with the predetermined laser ablation mode in this cycle, and the laser ablation mode needs to be changed. As can be appreciated, if in a specific cycle, the conformity index between the real-time lesion region information and the expected lesion region information exceeds the first set threshold, such as 10%, the effectof this laser ablation is basically achieved, this cycle is a final cycle, and the laser ablation is ended. In this regard, in this cycle, the conformity index between the real-time lesion region information at a specific time and the expected lesion region information that corresponds to this specific time reaches, for certain, the third set threshold, such as 10%.

Optionally, the real-time lesion region information includes real-time temperature information of a lesion region, and the laser ablation method further includes: controlling, in response to that the real-time temperature information is greater than a preset temperature threshold, an optical fiber assembly for outputting the laser to be cooled until subsequent real-time temperature information is not greater than the preset temperature threshold.

In this embodiment, the temperature information of the lesion region may be obtained in real-time. In a case where the temperature is too high, the control host can controls a cooling assembly to cool the laser emitted by the optical fiber assembly, thereby reducing the temperature applied to the lesion region and ensuring that the lesion region remains at an appropriate temperature.

Optionally, the preset temperature threshold is determined by the initial lesion region information.

In this embodiment, the initial lesion region information can determine the type of the initial lesion region. For example, after marking the tissue, which may include a lesion, nerve fiber tracts, a brain partition and a blood vessel, the initial lesion region information is determined. The position and type of the lesion that requires ablation are identified, and the preset temperature threshold for the lesion is determined based on the type of the lesion, thereby providing enhanced targeting capability.

Optionally, the laser ablation method further includes: obtaining real-time temperature information, and obtaining a three-dimensional temperature distribution map of the real-time temperature information based on the three-dimensional model of the user.

In this embodiment, the three-dimensional temperature distribution map may be displayed in real-time to assist the technicians performing the ablation in obtaining the temperature information of the lesion region, thereby reducing the implementation risk of the ablation method.

Optionally, obtaining the real-time temperature information includes: obtaining the real-time temperature information through parallel imaging, where a scanning slice thickness is not less than 3 mm and not greater than 5 mm; or, obtaining the real-time temperature information through three-dimensional scanning; or, obtaining the real-time temperature information through thin slice scanning, where a scanning slice thickness is less than 3 mm.

In this embodiment, the real-time temperature information may be obtained through different scan types of the magnetic resonance imaging technology, including distribution map display on a surface of segmented regions. In the case of the parallel imaging, the scanning slice thickness may be around three millimeters. Alternatively, a three-dimensional temperature scanning of the relevant region may be performed, or thinner layers scanning may be used and calculation is performed by using an interpolation method, which further aids the implementation of the ablation control algorithm. Moreover, when technicians evaluate ablation results through temperature displays, three-dimensional displays help them observe the ablation results in all directions, not just in a scanning direction, thereby making the evaluation about the ablation results more accurate.

Optionally, the real-time lesion region information includes: a plurality of pieces of target region information, and the method further includes: determining the laser ablation mode corresponding to each target region information.

For example, a specific target region information may be matched with a corresponding laser ablation mode for ablation, and temperature control may be implemented in a peripheral region surrounding the target region information. Alternatively, the temperature in the peripheral region surrounding the target region information may be stored and used as a reference in the next implementation of the ablation method.

Optionally, the real-time lesion region information includes boundary information of a lesion region, and the method further includes: determining in real time whether the boundary information exceeds a preset boundary threshold.

In this embodiment, the real-time lesion region information includes the boundary information of the lesion region. The boundary information is obtained in real time to determine specific location information of the ablation at each time. Next, the location information of the ablation is compared with the preset boundary threshold to determine whether the ablation exceeds the preset boundary threshold. If yes, the ablation operation may be suspended to avoid unnecessary damage to a region outside the preset boundary threshold.

Optionally, the method further includes: color-marking the real-time lesion region information in a three-dimensional model, where the real-time lesion region information corresponding to each time has a different marking color from the expected lesion region information.

In this embodiment, the real-time lesion region information being ablated may be displayed in one color, while the expected lesion region information may be displayed in another color, so that it is intuitive to determine whether the effect of this ablation has been achieved. Moreover, if there are regions that have undergone multiple ablations, multiple colors may also be displayed for warning purposes.

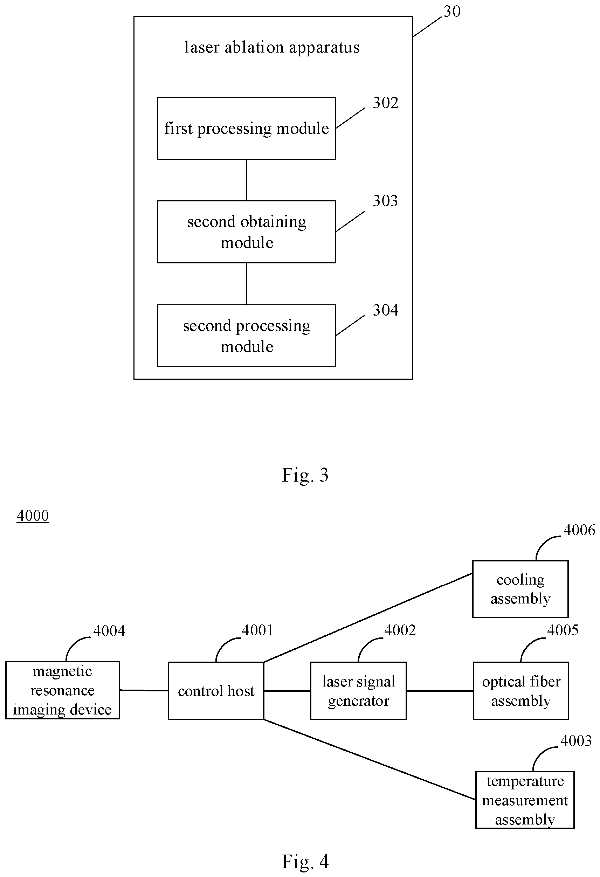

Based on the same inventive concept, the embodiments of the present disclosure provide a laser ablation apparatus 30, as shown in Fig. 3, including:
a first processing module 302, configured to determine a corresponding laser ablation mode based on real-time lesion region information at a first time;
a second obtaining module 303, configured to obtain real-time lesion region information at a (i+1)th time, wherein the (i+1)th time is later than an i-th time, and i is a positive integer; where a lesion region at the (i+1)th time is ablated based on a laser ablation mode at the i-th time; and
a second processing module 304, configured to determine a (i+1)th laser ablation mode according to a laser ablation strategy and the real-time lesion region information at the (i+1)th time, until a conformity index between real-time lesion region information at a subsequent time and expected lesion region information after a final laser ablation exceeds a first set threshold.

Optionally, the laser ablation apparatus further includes a first obtaining module 301, configured to obtain initial lesion region information of a user and a corresponding laser ablation strategy, where the laser ablation strategy includes a quantity of laser ablation, an expected laser ablation mode corresponding to each laser ablation, and expected lesion region information after each laser ablation.

Optionally, the laser ablation apparatus further includes a receiving module, configured to:
receive digital image information of the user;
perform multimodal three-dimensional modeling based on the digital image information to obtain a three-dimensional model of the user;
receive a marking operation for a position of a tissue in the three-dimensional model to determine the initial lesion region information of the user; where the tissue includes a lesion, a nerve and a blood vessel;
receive expected lesion region information after a target ablation of a lesion corresponding to the initial lesion region information.

Optionally, the laser ablation apparatus further includes a third processing module, configured to determine the laser ablation strategy based on the initial lesion region information and the expected lesion region information.

Optionally, the laser ablation apparatus further includes a third obtaining module, configured to obtain the real-time lesion region information at the first time.

Optionally, the laser ablation apparatus further includes a fourth processing module, configured to determine an expected laser ablation mode of a first laser ablation in the laser ablation strategy as the laser ablation mode in response to that a conformity index between the real-time lesion region information at the first time and the initial lesion region information is less than a second set threshold.

Optionally, the laser ablation apparatus further includes a fifth processing module, configured to
adjust, in response to that a conformity index between the expected lesion region information at the (i+1)th time in the laser ablation strategy and the real-time lesion region information at the (i+1)th time is less than a third set threshold, the expected laser ablation mode of the (i+1)th laser ablation according to the real-time lesion region information at the (i+1)th time, to obtain the corresponding laser ablation mode;
maintain, in response to that the conformity index between the expected lesion region information at the (i+1)th time and the real-time lesion region information at the (i+1)th time is greater than or equal to the third set threshold, a laser ablation mode at the i-th time as the laser ablation mode at the (i+1)th time.

Based on the same inventive concept, the embodiments of the present disclosure provide a laser ablation device 4000, including a control host 4001 and a computer program stored on the control host 4001, where the control host 4001 is configured to execute the computer program to implement the steps of the laser ablation method according to any one of the above mentioned embodiments.

As can be appreciated by a person skilled in the art, the laser ablation device 4000 in the embodiments of the present disclosure may be specially designed and manufactured for the required purpose, or it may also a known device including a general-purpose computer. These devices have computer programs stored therein, the computer programs are selectively activated or reconfigured. Such computer programs may be stored in a device (e.g., a computer) computer-readable medium or any type of medium appropriate for storing electronic instructions and coupled to a bus.

Optionally, the laser ablation device 4000 includes a magnetic resonance imaging device 4004, an optical fiber assembly 4005, a memory, a temperature measurement assembly 4003, a cooling assembly 4006, etc. The laser ablation device 4000 further includes a computer program stored in the memory, a laser signal generator 4002 to transmit a laser signal to the optical fiber assembly 4005, thereby controlling the optical fiber assembly to output an optical signal.

Optionally, before laser ablation, the control host 4001 of the laser ablation device 4000 may complete registration and three-dimensional modeling of a patient based on the digital image information provided by the magnetic resonance imaging device 4004.

Optionally, during the laser ablation, the control host 4001 of the laser ablation device 4000 may process the information transmitted by the magnetic resonance imaging device 4004 in a timely manner, generate the real-time temperature image, and display it on a human-computer interaction interface. In addition, based on feedback information from the human-computer interaction interface, it controls the cooling assembly 4006 and a plurality of optical fiber assemblies 4005, to manage the generation or power adjustment of various lasers, and drive and control the cooling assembly 4006 to regulate the temperature of the optical fiber assemblies 4005.

Optionally, the optical fiber assembly 4005 has a laser emission end and a cooling liquid return structure. A small diameter of an optical fiber, in conjunction with images provided by the magnetic resonance imaging device 4004, allows it to avoid brain nerves and penetrate into the cranial cavity for precise shape-following ablation of the lesion region (such as a tumor). By configuring multiple wavelengths and different types of optical fiber catheters, precise shape-following ablation may also be achieved for an irregular lesion region (such as tumor) without damaging normal tissue. For example, two wavelengths of laser may be emitted simultaneously from the optical fiber or sequentially from their respective fibers, or the same wavelength laser may be used two or more times to achieve ablation of the irregular shape.

Optionally, the laser ablation device 4000 further includes the temperature measurement assembly 4003, which may provide real-time temperature feedback to the control host 4001, so that the control host 4001 adjusts the temperature of the laser.

In the embodiments of the present disclosure, it is able to achieve effective ablation of the lesion region in the cranial cavity and output laser through different laser ablation modes to precisely adapt to irregular lesion regions. During the ablation process, the situation is monitored in real-time by the magnetic resonance device to avoid damage to normal tissue.

Optionally, the laser ablation device 4000 further includes the human-computer interaction interface, a power source module, and other peripheral interfaces.

Optionally, the human-computer interaction interface is used in conjunction with the control host 4001, including two 24-inch touch displays, one 10-inch touch display, physical buttons, and working indicators for display. It also provides input manners such as touchscreen, mouse, keyboard, and physical knobs. An emergency stop control switch may shut down various functional components but does not turn off the human-computer interaction interface.

Optionally, to enhance the stability and safety of the system, a control interface for the optical fiber assembly 4005 and the cooling assembly 4006 is equipped with two sets of foot switches to control laser emission. In a case where one set fails, the other can be used for control.

Optionally, in the embodiments of the present disclosure, a water-cooling manner, which has a stable cooling source, is effective, and has low in preparation cost, may be used. It includes in structure a peristaltic pump, a cooling liquid container, a waste liquid container, a cooling pipe, and cooling liquid. A single-channel system may be used to prevent contamination from circulation backflow. The cooling liquid container is equipped with a heater, a temperature sensor, and a liquid sensor. When the cooling assembly 4006 operates, the cooling liquid in the cooling liquid container is heated by a heating module to an appropriate temperature as detected by the temperature sensor. Next, the peristaltic pump begins to operate, delivering the cooling liquid into the optical fiber assembly 4005 to form a cooling circuit. Next, the cooling liquid enters the waste liquid container through the return pipe, with a flow sensor before the waste liquid container to confirm whether the cooling liquid is returning normally. The liquid sensor on the cooling liquid container may detect the liquid condition in the container, and when insufficient, an alarm signal is issued to request the replacement of the cooling liquid.

The optical fiber assembly 4005 may include at least two sub optical fiber units. One uses a 980-nanometer semiconductor laser unit capable of emitting high-power laser and has its own heat dissipation to ensure long-term stable output. Another one uses a 1064-nanometer Nd:YAG (a laser crystal material is yttrium-doped aluminum oxide) laser unit, capable of emitting high-power laser. Other laser units may also generate other forms of laser, which are not limited herein.

Optionally, other peripheral interfaces include, but are not limited to, USB (Universal Serial Bus) interface, safety switch interface, network cable, optical drive, etc.

Optionally, the optical fiber assembly 4005 includes an optical fiber catheter assembly and an optical fiber catheter securing assembly. The optical fiber catheter assembly is used for tumor ablation. The optical fiber catheter securing assembly is secured to the cranium of the patient, used to secure and guide the optical fiber catheter assembly, ensuring precise delivery of the optical fiber catheter to the lesion.

Optionally, the optical fiber catheter assembly is equipped with a dedicated cooling pipeline, where an outer pipe, inner pipe, ablation fiber, and other sealing members form a unidirectional cooling pipeline. Cooling water flows through the light-emitting position of the ablation fiber, thereby providing effective cooling for the ablation fiber. Thus, precise temperature control of the optical fiber catheter is achieved through adjustments by the control host 4001. A head end of the ablation fiber at the end of the optical fiber catheter penetrates into a brain to reach the lesion region for treatment. A laser emission end of the ablation fiber may be of various kinds, currently including diffusing, annular, and side-emitting, tailored for different lesion shapes. The ablation fiber, inner pipe, and outer pipe are mutually supported in structure, forming an independent cooling water path while also ensuring concentricity of the ablation fiber, inner pipe, and outer pipe. Regarding the optical fiber catheter securing assembly, it has at least one structure secured to the cranium, preferably a hollowed-out cranial screw. It further includes a structure for securing the optical fiber catheter assembly and provides guidance. The cranial screw has self-tapping threads that can secure it to the cranial. Next, the entire the optical fiber catheter securing assembly attaches to the cranial screw, maintaining a fixed position relative to a cranium of a patient. A hollowed-out inner hole of the cranial screw forms a guided passage with a position adjustment structure, directing the entry of the optical fiber catheter assembly.

Based on the same inventive concept, the embodiments of the present disclosure provide a computer readable storage medium having a computer program stored thereon, the computer program implementing, when executed by a laser ablation device, the steps of the laser ablation method according to any one of the above mentioned embodiments.

The computer readable storage medium in the embodiments of the present disclosure is applicable to any of various optional embodiments of the above laser ablation method, which will not be elaborated herein.

Based on the same inventive concept, the embodiments of the present disclosure further provide a computer program product, including a computer program, where the computer program implements, when executed by a control host, the steps of the laser ablation method according to any one of the above mentioned embodiments.

As can be appreciated by a person skilled in the art, steps, measures and schemes in various operations, methods and processes that have already been discussed in the embodiments of the present disclosure may be replaced, modified, combined or deleted. Further, the other steps, measures and schemes in various operations, methods and processes that have already been discussed in the embodiments of the present disclosure may also be replaced, modified, rearranged, decomposed, combined or deleted. Further, steps, measures and schemes in various operations, methods and processes that are known in the related art and have already been discussed in the embodiments of the present disclosure may also be replaced, modified, rearranged, decomposed, combined or deleted.

Such words as "first" and "second" are merely for illustrative purposes, rather than to implicitly or explicitly indicate the number of the defined technical features. In this regard, the technical features defined with such words as "first" and "second" may implicitly or explicitly include one or more technical features. Further, such a phrase as "a plurality of" is used to indicate that there are at least two, e.g., two or three, components, unless otherwise specified.

The above embodiments are only a part of the embodiments of the present disclosure, it should be appreciated that for those skilled in the art, other similar implementation manners based on the technical ideas of the present disclosure also fall within the scope of the embodiments of the present disclosure, provided they do not depart from the technical concept of the solution in the present disclosure.

## Claims

1. A laser ablation method, comprising:
determining a corresponding laser ablation mode based on real-time lesion region information at a first time;
obtaining real-time lesion region information at a (i+1)th time, wherein the (i+1)th time is later than an i-th time, and i is a positive integer;
determining whether a conformity index between the real-time lesion region information at the (i+1)th time and expected lesion region information after an i-th laser ablation exceeds a first set threshold;
determining, in response to that the conformity index between the real-time lesion region information at the (i+1)th time and the expected lesion region information after the i-th laser ablation exceeds the first set threshold, a (i+1)th laser ablation mode according to an expected laser ablation mode of a (i+1)th laser ablation in a laser ablation strategy and the real-time lesion region information at the (i+1)th time, and continuing to determine a next laser ablation mode through repeating the above steps of obtaining and subsequent determining;
ending the laser ablation in response to that the conformity index between the real-time lesion region information at the (i+1)th time and the expected lesion region information after the i-th laser ablation does not exceed the first set threshold.

2. The laser ablation method according to claim 1, wherein, prior to determining the corresponding laser ablation mode based on the real-time lesion region information at the first time, the method further comprises:
obtaining initial lesion region information of a user and a corresponding laser ablation strategy, wherein the laser ablation strategy comprises a quantity of laser ablation, an expected laser ablation mode corresponding to each laser ablation, and expected lesion region information after each laser ablation.

3. The laser ablation method according to claim 2, wherein, prior to obtaining the initial lesion region information of the user and the corresponding laser ablation strategy, the method further comprises:
receiving digital image information of the user;
performing multimodal three-dimensional modeling based on the digital image information to obtain a three-dimensional model of the user;
receiving a marking operation for a position of a tissue in the three-dimensional model to determine the initial lesion region information of the user; wherein the tissue comprises a lesion, nerve fiber tracts, a brain partition and a blood vessel;
receiving expected lesion region information after a target ablation of a lesion corresponding to the initial lesion region information; and
determining the laser ablation strategy based on the initial lesion region information and the expected lesion region information.

4. The laser ablation method according to claim 3, further comprising:
receiving the marking operation for the position of the tissue in the three-dimensional model,
determining whether there is a non-ablation region, and
marking the non-ablation region in response to that there is a non-ablation region.

5. The laser ablation method according to claim 3, wherein determining the corresponding laser ablation mode based on the real-time lesion region information at the first time comprises:
obtaining the real-time lesion region information at the first time; and
determining an expected laser ablation mode of a first laser ablation in the laser ablation strategy as the laser ablation mode in response to that a conformity index between the real-time lesion region information at the first time and the initial lesion region information is less than a second set threshold.

6. The laser ablation method according to claim 1, wherein determining the (i+1)th laser ablation mode according to the expected laser ablation mode of the (i+1)th laser ablation in the laser ablation strategy and the real-time lesion region information at the (i+1)th time comprises:
adjusting, in response to that a conformity index between the expected lesion region information at the (i+1)th time in the laser ablation strategy and the real-time lesion region information at the (i+1)th time is less than a third set threshold, the expected laser ablation mode of the (i+1)th laser ablation according to the real-time lesion region information at the (i+1)th time, to obtain the corresponding laser ablation mode;
maintaining, in response to that the conformity index between the expected lesion region information at the (i+1)th time and the real-time lesion region information at the (i+1)th time is greater than or equal to the third set threshold, a laser ablation mode at the i-th time as the laser ablation mode at the (i+1)th time.

7. The laser ablation method according to claim 2, wherein the real-time lesion region information comprises real-time temperature information of a lesion region; and the laser ablation method further comprises:
controlling, in response to that the real-time temperature information is greater than a preset temperature threshold, an optical fiber assembly for outputting the laser to be cooled until subsequent real-time temperature information is not greater than the preset temperature threshold;
wherein the laser ablation method further comprises:
obtaining real-time temperature information, and obtaining a three-dimensional temperature distribution map of the real-time temperature information based on the three-dimensional model of the user.

8. The laser ablation method according to claim 10, wherein obtaining the real-time temperature information comprises:
obtaining the real-time temperature information through parallel imaging, wherein a scanning slice thickness is not less than 3 mm and not greater than 5 mm; or,
obtaining the real-time temperature information through three-dimensional scanning; or,
obtaining the real-time temperature information through thin slice scanning, wherein a scanning slice thickness is less than 3 mm.

9. The laser ablation method according to claim 1, wherein the real-time lesion region information comprises: a plurality of pieces of target region information, and the method further comprises:
determining the laser ablation mode corresponding to each target region information.

10. The laser ablation method according to claim 1, wherein the real-time lesion region information comprises boundary information of a lesion region, and the method further comprises:
determining in real time whether the boundary information exceeds a preset boundary threshold.

11. The laser ablation method according to claim 1, further comprising:
displaying, in real time, the real-time lesion region information at each time and the conformity index between the real-time lesion region information at each time and the expected lesion region information through a three-dimensional model.

12. The laser ablation method according to claim 1, further comprising:
color-marking the real-time lesion region information in a three-dimensional model, wherein the real-time lesion region information corresponding to each time has a different marking color from the expected lesion region information.

13. A laser ablation apparatus, comprising:
a first processing module, configured to determine a corresponding laser ablation mode based on real-time lesion region information at a first time;
a second obtaining module, configured to obtain real-time lesion region information at a (i+1)th time, wherein the (i+1)th time is later than an i-th time, and i is a positive integer; wherein a lesion region at the (i+1)th time is ablated based on a laser ablation mode at the i-th time; and
a second processing module, configured to determine a (i+1)th laser ablation mode according to a laser ablation strategy and the real-time lesion region information at the (i+1)th time, until a conformity index between real-time lesion region information at a subsequent time and expected lesion region information after a final laser ablation exceeds a first set threshold.

14. A laser ablation device, comprising a control host and a computer program stored on the control host, wherein the control host is configured to execute the computer program to implement the steps of the laser ablation method according to any one of claims 1 to 12.

15. A computer program product, comprising a computer program, wherein the computer program implements, when executed by a control host, the steps of the laser ablation method according to any one of claims 1 to 12.
